# EUROPEAN PATENT SPECIFICATION

(11) **EP 4 015 024 B1**
(45) Date of publication and mention of the grant of the patent: **13.03.2024**
(21) Application number: 21213107.2
(22) Date of filing: 08.12.2021
(51) Int. Cl.: A61M 16/04, A61B 1/267

(54) **ENDOTRACHEAL TUBE**
ENDOTRACHEALTUBUS
TUBE ENDOTRACHÉAL

(30) Priority: 16.12.2020 TW 109144561
(43) Date of publication of application: 22.06.2022
(73) Proprietor: Chen, Tien-Sheng, Taipei City 112 (TW)
(72) Inventor: Chen, Tien-Sheng, Taipei City 112 (TW)
(74) Representative: Viering, Jentschura & Partner mbB Patent- und Rechtsanwälte

(56) References cited:
- WO-A1-2016/044438
- US-A1- 2002 195 110
- US-A1- 2017 232 216

## Description

### BACKGROUND OF THE INVENTION

### 1. Field of the Invention

The present invention relates to an endotracheal tube; more particularly, the present invention relates to an endotracheal tube having a tip which can be turned to face toward the center of the trachea upon movement of a laryngoscope blade so as to increase the success rate of endotracheal intubation.

### 2. Description of the Related Art

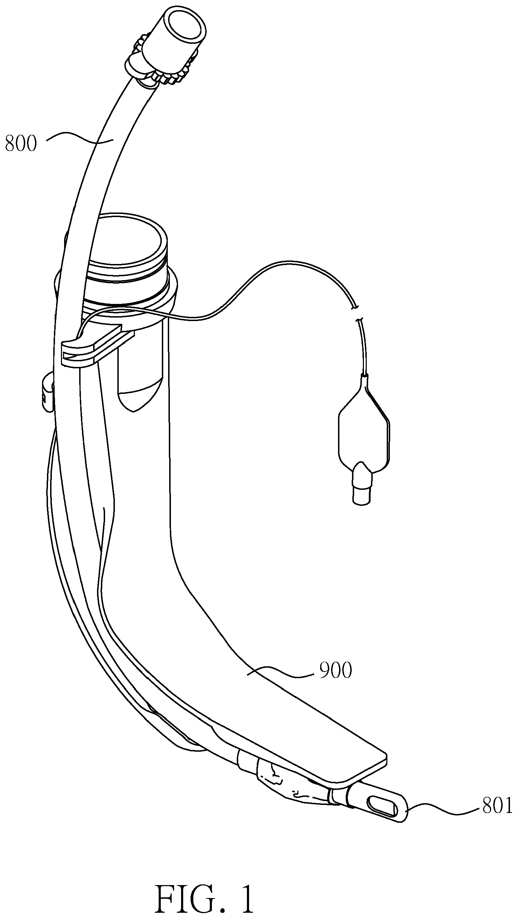

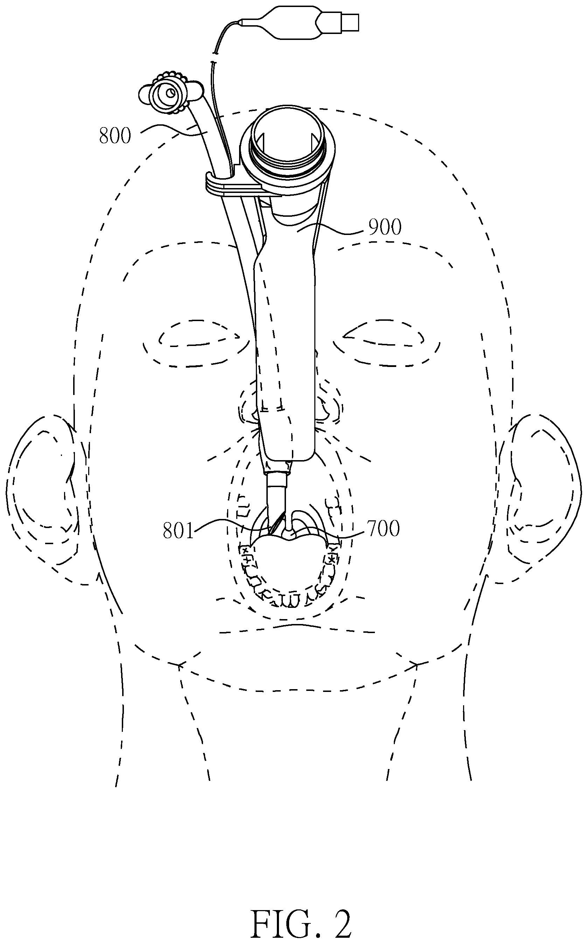

Please refer to FIG. 1 and FIG. 2, which illustrate a conventional endotracheal tube 800 having a conventional endotracheal tube tip 801 commonly used in the current medical profession. When a doctor performs endotracheal intubation on a patient, the conventional endotracheal tube 800 needs to be placed onto a laryngoscope blade 900, and then the laryngoscope blade 900 is inserted into the patient's trachea. The width of such a laryngoscope blade 900 is about 2.2 to 2.4 cm. Ideally, if the doctor wants to ensure that the conventional endotracheal tube 800 placed onto the right side of the laryngoscope blade 900 faces toward the center of the patient's tracheal passage 700, the width of the patient's larynx needs to be greater than 4.4 to 4.8 cm. However, the width of some adults' larynxes is smaller than 4 cm. Therefore, under the condition that the width of the patient's larynx is smaller than 4 cm, it is not feasible for the existing laryngoscope blade 900 to cause the conventional endotracheal tube 800, which has the conventional endotracheal tube tip 801 oblique from the right side to the left side, to face toward the center of the patient's tracheal passage 700. As a result, the failure rate of endotracheal intubation using such a laryngoscope is still high. Also, because the conventional endotracheal tube tip 801 is oblique from the right side to the left side, then if the conventional endotracheal tube tip 801 cannot face toward the center of the patient's tracheal passage 700, the conventional endotracheal tube tip 801 can easily cause soft tissue injury to the larynx when the doctor moves the conventional endotracheal tube 800.

Further, for those patients having a smaller throat diameter, the doctor's operable width of the larynx will be even narrower. Under such circumstances, it is more difficult for the conventional endotracheal tube tip 801 to face toward the center of the patient's trachea, thereby increasing the difficulty of endotracheal intubation. However, because the laryngoscope is a commonly used medical device in the current medical profession, there is a need to improve the design of the conventional endotracheal tube 800 itself so as to increase the success rate of endotracheal intubation using the laryngoscope, as well as to reduce the risk of injury to the soft tissue of the patient's larynx caused by the conventional endotracheal tube tip 801.

Therefore, there is a need to provide an endotracheal tube to mitigate and/or obviate the aforementioned problems.

Further, US 2002/195110 A1 discloses an endotracheal tube, comprising a curved tube body and an oblique cutting surface, the curved tube body having a front end and the curved tube body having a center axis, an inner axis and an outer axis , the oblique cutting surface having a tip and the oblique cutting surface being located at the front end, wherein the oblique cutting surface has a circular projection surface on a vertical plane, the tip has a tip projection point on the circumference of the circular projection surface, the inner axis has an inner axis projection point on the circumference of the circular projection surface

### SUMMARY OF THE INVENTION

It is an object of the present invention to provide an endotracheal tube capable of increasing the success rate of endotracheal intubation via guidance of an endotracheal tube tip to face toward the center of a tracheal passage upon movement of a laryngoscope blade when the width of a larynx is limited.

To achieve the abovementioned object, the present invention provides the endotracheal tube according to claim 1. The dependent claims show further preferred embodiments of the said endotracheal tube.

The endotracheal tube of the present invention comprises a curved tube body and an oblique cutting surface. The curved tube body has a front end, and the curved tube body has a center axis, an inner axis and an outer axis. The oblique cutting surface has a tip, and the oblique cutting surface is located at the front end. The endotracheal tube has the following characteristics: the oblique cutting surface has a circular projection surface on a vertical plane, the tip has a tip projection point on the circumference of the circular projection surface, the inner axis has an inner axis projection point on the circumference of the circular projection surface, and the tip projection point and the inner axis projection point form an angle θ, where 15° < θ < 45°.

Because the cutting direction of the oblique cutting surface located at the front end of the endotracheal tube of the present invention is oblique from a right side tube portion to a left side tube portion, the endotracheal tube tip can therefore face toward the center of a patient's tracheal passage if the width of the patient's larynx is smaller than 4 cm, and this ability will increase the success rate of endotracheal intubation, especially by increasing the success rate of endotracheal intubation on patients having a smaller oral cavity or narrower larynx, which complicate endotracheal intubation, and this design also reduces the risk of soft tissue injury caused by the endotracheal tube tip.

Other objects, advantages, and novel features of the invention will become more apparent from the following detailed description when taken in conjunction with the accompanying drawings.

### BRIEF DESCRIPTION OF THE DRAWINGS

These and other objects and advantages of the present invention will become apparent from the following description of the accompanying drawings, which disclose several embodiments of the present invention. It is to be understood that the drawings are to be used for purposes of illustration only and not as a definition of the invention.

In the drawings, wherein similar reference numerals denote similar elements throughout the several views:
FIG. 1 illustrates a schematic drawing of a conventional endotracheal tube placed onto a laryngoscope blade.
FIG. 2 illustrates a schematic drawing showing corresponding positions of the conventional endotracheal tube placed onto the laryngoscope blade after being inserted into a human trachea.
FIG. 3 illustrates a schematic drawing of an endotracheal tube placed onto a laryngoscope blade according to a first embodiment of the present invention.
FIG. 4A illustrates a schematic drawing of the endotracheal tube according to the first embodiment of the present invention.
FIG. 4B illustrates a schematic drawing showing a circular projection surface on a vertical plane relative to an oblique cutting surface of the endotracheal tube according to the present disclosure, wherein only angles θ within the range of 15° < θ < 45° are covered by the claim set.
FIG. 5 illustrates a cross-sectional schematic drawing of a front end of the endotracheal tube according to the first embodiment of the present invention.
FIG. 6 illustrates a schematic drawing showing corresponding positions of the endotracheal tube placed onto the laryngoscope blade after being inserted into a human trachea according to the present invention.
FIG. 7 illustrates a right side view of the endotracheal tube according to a second embodiment of the present invention, wherein the second embodiment is not showing all features of claim 1, but is provided as additional information.
FIG. 8 illustrates a left side view of the endotracheal tube according to the second embodiment of the present invention, wherein the second embodiment is not showing all features of claim 1, but is provided as additional information.
FIG. 9 illustrates a top view of the endotracheal tube according to the second embodiment of the present invention, wherein the second embodiment is not showing all features of claim 1, but is provided as additional information.
FIG. 10 illustrates a bottom view of the endotracheal tube according to the second embodiment of the present invention, wherein the second embodiment is not showing all features of claim 1, but is provided as additional information.

### DETAILED DESCRIPTION OF THE PREFERRED EMBODIMENT

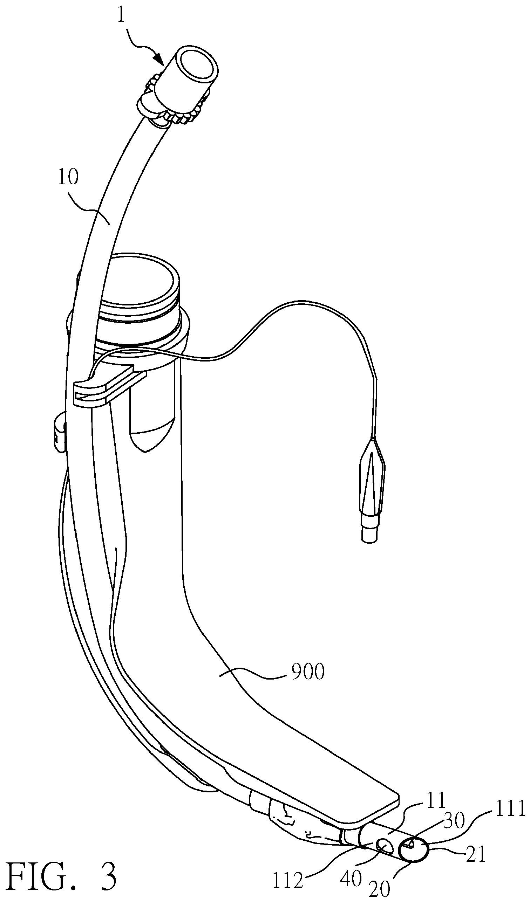

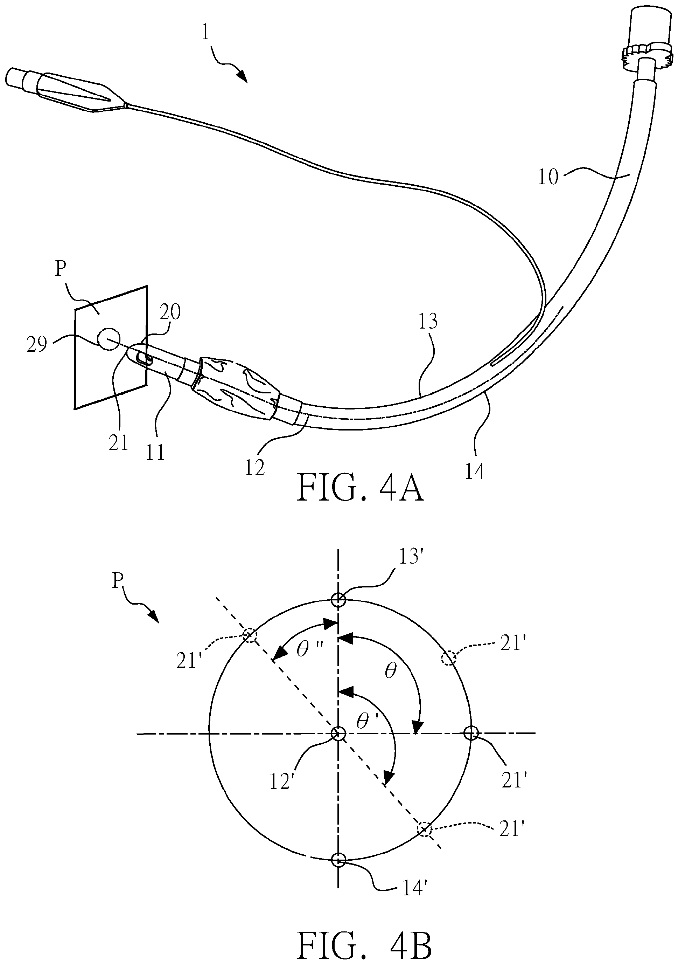

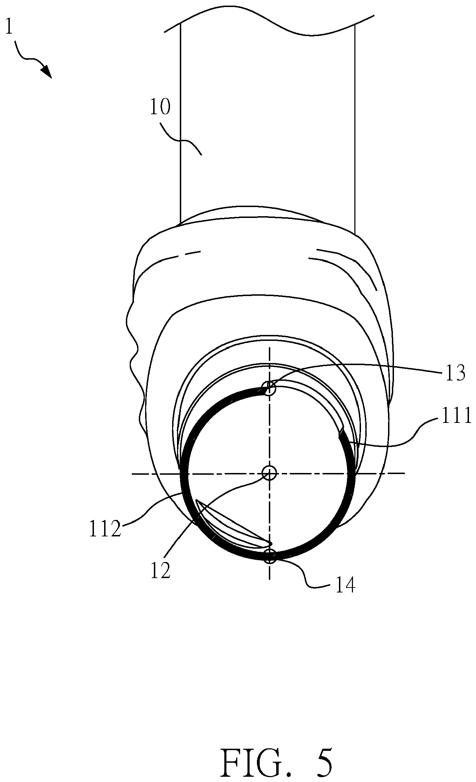

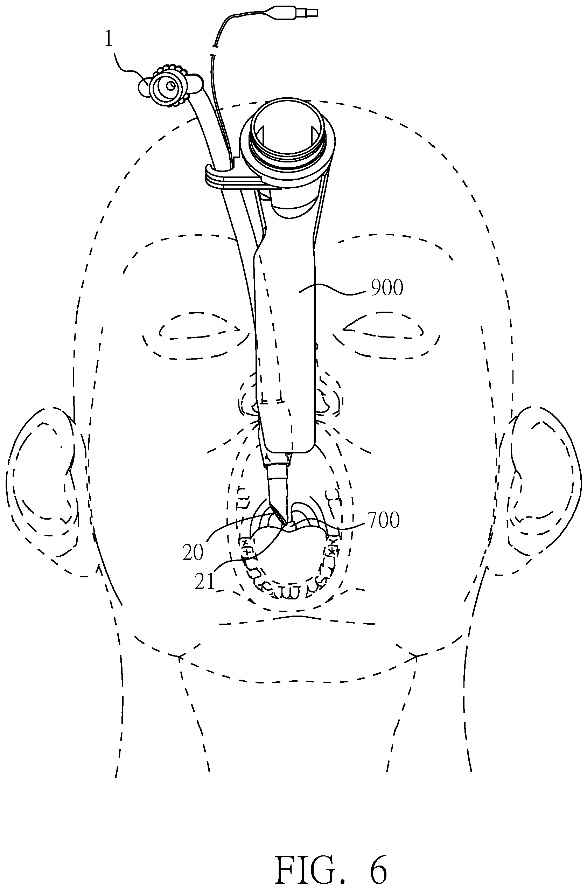

Please refer to FIG. 3 to FIG. 6, wherein FIG. 3 illustrates a schematic drawing of an endotracheal tube placed onto a laryngoscope blade according to a first embodiment of the present invention; FIG. 4A illustrates a schematic drawing of the endotracheal tube according to the first embodiment of the present invention; FIG. 4B illustrates a schematic drawing showing a circular projection surface on a vertical plane relative to an oblique cutting surface of the endotracheal tube according to the present invention; FIG. 5 illustrates a cross-sectional schematic drawing of a front end of the endotracheal tube according to the first embodiment of the present invention; and FIG. 6 illustrates a schematic drawing showing corresponding positions of the endotracheal tube placed onto the laryngoscope blade after being inserted into a human trachea according to the present invention.

As shown in FIG. 3, FIG. 4A and FIG. 4B, in the first embodiment, an endotracheal tube 1 of the present invention comprises a curved tube body 10 and an oblique cutting surface 20. The curved tube body 10 has a front end 11. The curved tube body 10 has a center axis 12, an inner axis 13 and an outer axis 14. The oblique cutting surface 20 has a tip 21, and the oblique cutting surface 20 is located at the front end 11. The endotracheal tube 1 of the present invention is characterized in that: the oblique cutting surface 20 has a circular projection surface 29 on a vertical plane P, the tip 21 has a tip projection point 21' on the circumference of the circular projection surface 29, the inner axis 13 has an inner axis projection point 13' on the circumference of the circular projection surface 29, and the tip projection point 21' and the inner axis projection point 13' form an angle θ, where -45° < θ < 90° , wherein only angles θ within the range of 15° < θ < 45° are covered by the claim set. By means of changing the direction of the tip 21 of the oblique cutting surface 20, as shown in FIG. 6, the tip 21 of the endotracheal tube 1 of the present invention may face toward the center of a patient's tracheal passage 700 if the width of the patient's larynx is smaller than 4 cm so as to increase the success rate of endotracheal intubation, especially by increasing the success rate of endotracheal intubation on those patients having a smaller oral cavity or narrower larynx, which increase the difficulty of endotracheal intubation, and also to reduce the risk of soft tissue injury caused by the tip 21. Please note that, as shown in FIG. 4B, on the circular projection surface 29, the inner axis projection point 13' is used as a starting point for defining moving right (i.e., moving along the clockwise direction) from the inner axis projection point 13' as being positive, and moving left (i.e., moving along the counterclockwise direction) from the inner axis projection point 13' as being negative. As a result, -45° means the tip projection point 21' is located to the left of the inner axis projection point 13', which is illustrated as angle θ" in FIG. 4B (provided as additional information; please note that only angles θ within the range of 15° < θ < 45° are covered by the claim set). Similarly, θ < 90° means the tip projection point 21' is located to the right of the inner axis projection point 13', which is illustrated as angle θ' in FIG. 4B (provided as additional information; please note that only angles θ within the range of 15° < θ < 45° are covered by the claim set). Please note that, as shown in FIG. 4A, the tip 21 of the endotracheal tube 1 of the first embodiment would have its tip projection point 21' projected onto the circumference of the circular projection surface 29 at the angle of θ = 90°; however, please note that the scope of the present invention is limited to 15° < θ < 45°.

Please refer to the center axis 12, the inner axis 13 and the outer axis 14 of the curved tube body 10 as shown in FIG. 4A and FIG. 5, wherein the center axis 12 is an extended line of the pipe center of the curved tube body 10, the inner axis 13 is an axis located at the side of the curved tube body 10 and has a higher curvature when the curved tube body 10 bends naturally, and the outer axis 14 is an axis located at the side of the curved tube body 10 and has a lower curvature when the curved tube body 10 bends naturally.

As shown in FIG. 3, FIG. 4A, FIG. 4, FIG. 5 and FIG. 6, the front end 11 comprises a right side tube portion 111 and a left side tube portion 112. According to the endotracheal tube 1 of the present invention, positions of the right side tube portion 111 and the left side tube portion 112 are defined based on the viewing angle of the cross-sectional schematic drawing of the front end 11 as shown in FIG. 5, wherein the right side of the cross-sectional schematic drawing is the right side tube portion 111, and the left side of the cross-sectional schematic drawing is the left side tube portion 112. The cutting direction of the oblique cutting surface 20 of the endotracheal tube 1 of the present invention is oblique from the right side tube portion 111 to the left side tube portion 112, which is different from the cutting direction of the conventional endotracheal tube 800, such that when the tip 21 of the endotracheal tube 1 of the present invention is used with the laryngoscope blade 900, the tip 21 of the endotracheal tube 1 of the present invention will be located at the center of the tracheal passage 700, as shown in FIG. 6, so as to increase the success rate of endotracheal intubation, as well as to effectively reduce the risk of soft tissue injury caused by the tip 21.

Please refer to FIG. 3, FIG. 4B and FIG. 5. The endotracheal tube 1 of the present invention comprises a first opening 30 and a second opening 40. The first opening 30 and the second opening 40 are both located on the front end 11 and are respectively located on two opposite sides of the tip 21. According to the arrangement of the first opening 30 and the second opening 40, oxygen may enter the patient's lung to maintain the patient's respiration if the oblique cutting surface 20 is blocked. In the first embodiment of the present invention, the first opening 30 is located at the right side tube portion 111 and the second opening 40 is located at the left side tube portion 112, wherein the first opening 30 is elliptical in shape and the second opening 40 is circular in shape. The first opening 30 has a first aperture 31, the second opening 40 has a second aperture 41, and the first aperture 31 is greater than the second aperture 41. According to one preferred embodiment of the present invention, the size of the second aperture 41 is, but is not limited to, 20% to 80% of the size of the first aperture 31.

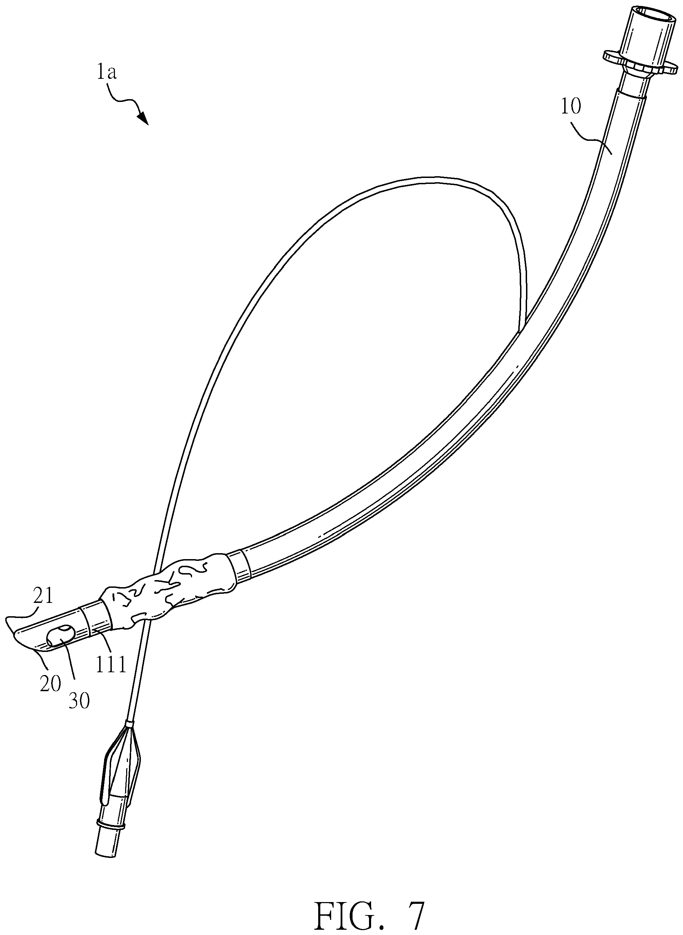

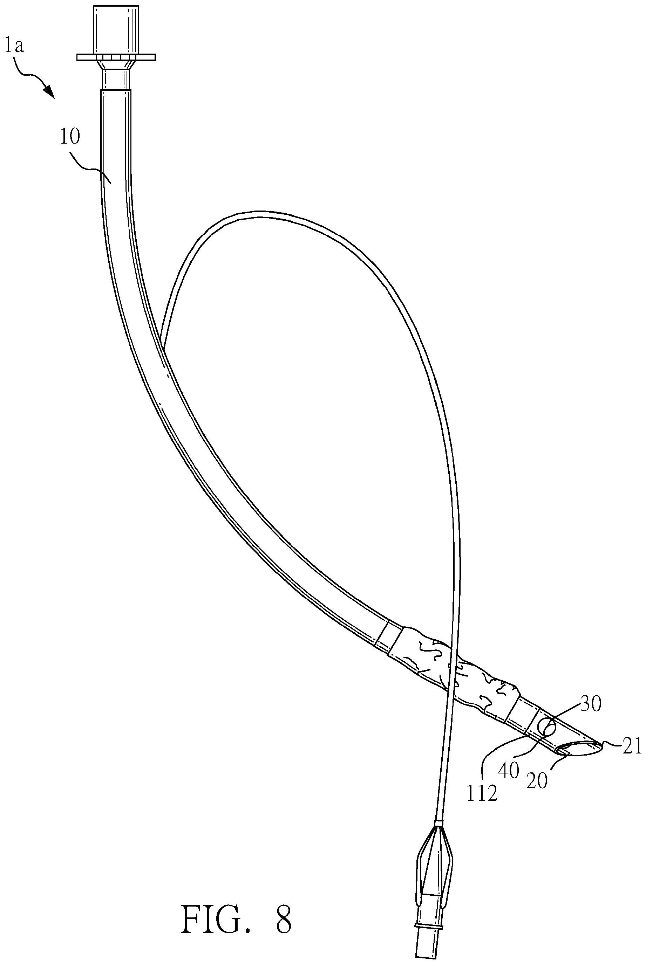

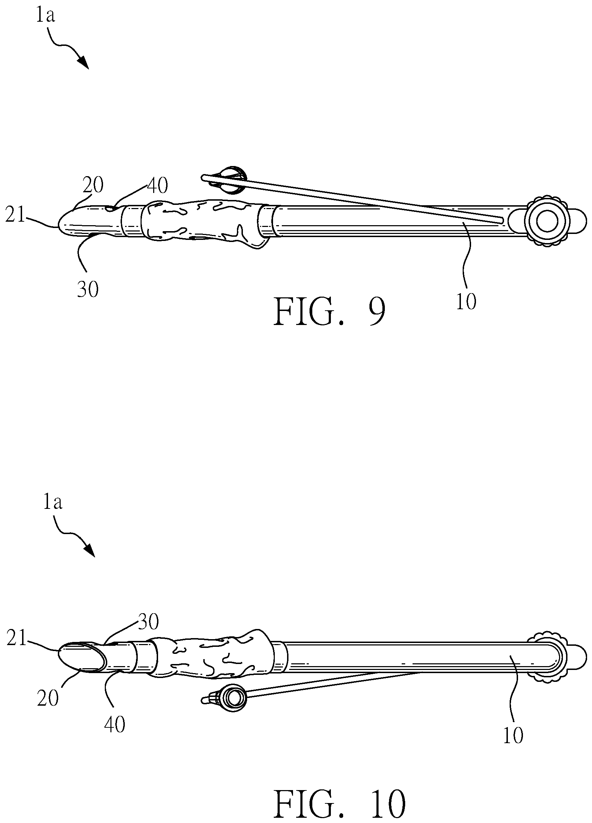

Please refer to FIG. 4B and FIGs. 7 to 10, which present the right side view, left side view, top view and bottom view of the endotracheal tube according to a second embodiment of the present invention, wherein the second embodiment is not showing all features of claim 1, but is provided as additional information.

As shown in FIG. 4B and FIGs. 7 to 10, the difference between the first embodiment and the second embodiment is that the tip 21 of the endotracheal tube 1a of the second embodiment has its tip projection point 21' projected onto the circumference of the circular projection surface 29 at the angle of θ = 0°. At this time, the first opening 30 and the second opening 40 are both located on the front end 11 and are respectively located on two opposite sides of the tip 21. The first opening 30 is located at the right side tube portion 111, and the second opening 40 is located at the left side tube portion 112.

The endotracheal tubes 1 and 1a of the present invention can make the tip 21 face toward the center of the patient's tracheal passage 700 upon movement of the laryngoscope blade 900 so as to increase the success rate of endotracheal intubation. Because the cutting direction of the oblique cutting surface 20 located at the front end 11 of the endotracheal tubes 1 and 1a of the present invention is oblique from the right side tube portion 111 to the left side tube portion 112, the tip 21 of the endotracheal tubes 1 and 1a can therefore face toward the center of the patient's tracheal passage 700 if the width of the patient's larynx is smaller than 4 cm so as to increase the success rate of endotracheal intubation, especially for those patients having a smaller oral cavity or narrower larynx, which increase the difficulty of endotracheal intubation, and also to reduce the risk of soft tissue injury caused by the tip 21.

Although the present invention has been explained in relation to its preferred embodiments, it is to be understood that many other possible modifications and variations can be made without departing from the scope of the invention as hereinafter claimed.

## Claims

1. An endotracheal tube (1), comprising a curved tube body (10) and an oblique cutting surface (20), the curved tube body (10) having a front end (11) and the curved tube body (10) having a center axis (12), an inner axis (13) and an outer axis (14), the oblique cutting surface (20) having a tip (21) and the oblique cutting surface (20) being located at the front end (11), wherein the oblique cutting surface (20) has a circular projection surface (29) on a vertical plane (P), the tip (21) has a tip projection point (21') on the circumference of the circular projection surface (29), the inner axis (13) has an inner axis projection point (13') on the circumference of the circular projection surface (29), and wherein the endotracheal tube (1) is **characterized in that**: the tip projection point (21') and the inner axis projection point (13') form an angle θ, where 15° < θ < 45°; wherein, on the circular projection surface (29), the inner axis projection point (13') is used as a starting point for defining moving along the clockwise direction from the inner axis projection point (13') as being positive, and moving along the counterclockwise direction from the inner axis projection point (13') as being negative.

2. The endotracheal tube (1) as claimed in claim 1, further comprising a first opening (30) and a second opening (40), wherein the first opening (30) and the second opening (40) are both located on the front end (11) and respectively located on two opposite sides of the tip (21).

3. The endotracheal tube (1) as claimed in claim 2, wherein the front end (11) further comprises a right side tube portion (111) and a left side tube portion (112), the first opening (30) being located on the right side tube portion (111) and the second opening (40) being located on the left side tube portion (112).

4. The endotracheal tube (1) as claimed in claim 1, wherein the endotracheal tube (1) comprises a first opening (30) and a second opening (40), and the front end (11) comprises a right side tube portion and a left side tube portion, the first opening and the tip being located on the right side tube portion and the second opening being located on the left side tube portion.

5. The endotracheal tube (1) as claimed in any of claims 2 to 4, wherein the first opening (30) has a first aperture (31), the second opening (40) has a second aperture (41), and the first aperture (31) is greater in size than the second aperture (41).

6. The endotracheal tube (1) as claimed in claim 5, wherein the size of the second aperture (41) is 20% to 80% of the size of the first aperture (31).

7. The endotracheal tube (1) as claimed in claim 6, wherein the first opening (30) is elliptical in shape and the second opening (40) is circular in shape.

## Patentansprüche

1. Endotrachealtubus (1), welcher einen gekrümmten Tubuskörper (10) und eine schräge Schneidfläche (20) aufweist, wobei der gekrümmte Tubuskörper (10) ein vorderes Ende (11) hat und der gekrümmte Tubuskörper (10) eine Mittellinie (12), eine Innenlinie (13) und eine Außenlinie (14) hat, wobei die schräge Schneidfläche (20) eine Spitze (21) hat und sich die schräge Schneidfläche (20) an dem vorderen Ende (11) befindet, wobei die schräge Schneidfläche (20) eine kreisförmige Projektionsfläche (29) an einer vertikalen Ebene (P) hat, die Spitze (21) einen Spitzenprojektionspunkt (21') an dem Umfang der kreisförmigen Projektionsfläche (29) hat, die Innenlinie (13) einen Innenlinienprojektionspunkt (13') an dem Umfang der kreisförmigen Projektionsfläche (29) hat, und wobei der Endotrachealtubus (1) **dadurch gekennzeichnet ist, dass**: der Spitzenprojektionspunkt (21') und der Innenlinienprojektionspunkt (13') einen Winkel θ bilden, wobei 15° < θ < 45°; wobei an der kreisförmigen Projektionsfläche (29) der Innenlinienprojektionspunkt (13') als ein Ausgangspunkt verwendet wird zum Definieren von Bewegung entlang der Uhrzeigersinn-Richtung von dem Innenlinienprojektionspunkt (13') aus als positiv seiend und von Bewegung entlang der Gegenuhrzeigersinn-Richtung von dem Innenlinienprojektionspunkt (13') aus als negativ seiend.

2. Endotrachealtubus (1) wie in Anspruch 1 beansprucht, welcher ferner eine erste Öffnung (30) und eine zweite Öffnung (40) aufweist, wobei sich die erste Öffnung (30) und die zweite Öffnung (40) beide an dem vorderen Ende (11) befinden und sich in jeweils zugeordneter Weise an zwei gegenüberliegenden Seiten von der Spitze (21) befinden.

3. Endotrachealtubus (1) wie in Anspruch 2 beansprucht, wobei das vordere Ende (11) ferner einen rechtsseitigen Tubusabschnitt (111) und einen linksseitigen Tubusabschnitt (112) aufweist, wobei sich die erste Öffnung (30) an dem rechtsseitigen Tubusabschnitt (111) befindet und sich die zweite Öffnung (40) an dem linksseitigen Tubusabschnitt (112) befindet.

4. Endotrachealtubus (1) wie in Anspruch 1 beansprucht, wobei der Endotrachealtubus (1) eine erste Öffnung (30) und eine zweite Öffnung (40) aufweist, und das vordere Ende (11) einen rechtsseitigen Tubusabschnitt und einen linksseitigen Tubusabschnitt aufweist, wobei sich die erste Öffnung und die Spitze an dem rechtsseitigen Tubusabschnitt befinden und sich die zweite Öffnung an dem linksseitigen Tubusabschnitt befindet.

5. Endotrachealtubus (1) wie in einem von den Ansprüchen 2 bis 4 beansprucht, wobei die erste Öffnung (30) eine erste Öffnungsweite (31) aufweist, die zweite Öffnung (40) eine zweite Öffnungsweite (41) aufweist, und die erste Öffnungsweite (31) größenmäßig größer als die zweite Öffnungsweite (41) ist.

6. Endotrachealtubus (1) wie in Anspruch 5 beansprucht, wobei die Größe der zweiten Öffnungsweite (41) 20% bis 80% der Größe der ersten Öffnungsweite (31) beträgt.

7. Endotrachealtubus (1) wie in Anspruch 6 beansprucht, wobei die erste Öffnung (30) in Gestalt elliptisch ist und die zweite Öffnung (40) in Gestalt kreisförmig ist.

## Revendications

1. Tube endotrachéal (1), comprenant un corps de tube incurvé (10) et une surface de coupe oblique (20), le corps de tube incurvé (10) ayant une extrémité avant (11) et le corps de tube incurvé (10) ayant un axe central (12), un axe intérieur (13) et un axe extérieur (14), la surface de coupe oblique (20) ayant une pointe (21) et la surface de coupe oblique (20) étant située à l'extrémité avant (11), dans lequel la surface de coupe oblique (20) présente une surface de projection circulaire (29) sur un plan vertical (P), la pointe (21) présente un point de projection de pointe (21') sur la circonférence de la surface de projection circulaire (29), l'axe intérieur (13) présente un point de projection d'axe intérieur (13') sur la circonférence de la surface de projection circulaire (29), et dans lequel le tube endotrachéal (1) est **caractérisé en ce que** : le point de projection de pointe (21') et le point de projection d'axe intérieur (13') forment un angle θ, où 15° < θ < 45° ; dans lequel, sur la surface de projection circulaire (29), le point de projection d'axe intérieur (13') est utilisé comme point de départ pour définir un déplacement dans le sens des aiguilles d'une montre à partir du point de projection d'axe intérieur (13') comme étant positif, et un déplacement dans le sens inverse des aiguilles d'une montre à partir du point de projection d'axe intérieur (13') comme étant négatif.

2. Tube endotrachéal (1) selon la revendication 1, comprenant en outre une première ouverture (30) et une deuxième ouverture (40), la première ouverture (30) et la deuxième ouverture (40) étant toutes les deux situées sur l'extrémité avant (11) et respectivement situées sur deux côtés opposés de la pointe (21).

3. Tube endotrachéal (1) selon la revendication 2, dans lequel l'extrémité avant (11) comprend en outre une partie de tube latérale droite (111) et une partie de tube latérale gauche (112), la première ouverture (30) étant située sur la partie de tube latérale droite (111) et la deuxième ouverture (40) étant située sur la partie de tube latérale gauche (112).

4. Tube endotrachéal (1) selon la revendication 1, dans lequel le tube endotrachéal (1) comprend une première ouverture (30) et une deuxième ouverture (40), et l'extrémité avant (11) comprend une partie de tube latérale droite et une partie de tube latérale gauche, la première ouverture et la pointe étant situées sur la partie de tube latérale droite et la deuxième ouverture étant située sur la partie de tube latérale gauche.

5. Tube endotrachéal (1) selon l'une quelconque des revendications 2 à 4, dans lequel la première ouverture (30) présente une première aperture (31), la deuxième ouverture (40) présente une deuxième aperture (41), et la première aperture (31) est de taille supérieure à la deuxième aperture (41) .

6. Tube endotrachéal (1) selon la revendication 5, dans lequel la taille de la deuxième aperture (41) est de 20 % à 80 % de la taille de la première ouverture (31).

7. Tube endotrachéal (1) selon la revendication 6, dans lequel la première ouverture (30) est de forme elliptique et la deuxième ouverture (40) est de forme circulaire.
